# EUROPEAN PATENT APPLICATION

(11) **EP 4 815 073 A1**
(43) Date of publication of application: **30.09.2026**
(21) Application number: 26165793.6
(22) Date of filing: 18.03.2026
(51) Int. Cl.: H01M 8/04089, C01B 3/025, C07C 29/152, C10G 2/00, C21B 13/00, C25B 1/23, H01M 8/0612, H01M 8/0668, H01M 8/10, H01M 8/249

(54) **SYSTEM AND METHOD FOR PROCESSING SYNGAS FROM FUEL CELL SYSTEM EXHAUST**

(30) Priority: 28.03.2025 US 202563779740 P
(71) Applicant: Bloom Energy Corporation, San Jose, CA 95134 (US)
(72) Inventor: WEINGAERTNER, David, San Jose, CA 95134 (US); KETKAEW, Jittisa, San Jose, CA 95134 (US)
(74) Representative: Wichmann, Hendrik

(57) **Abstract**

A method includes providing fuel and air to a fuel cell system to generate electric power, an anode exhaust and a cathode exhaust, separating at least a portion of water and carbon dioxide from the anode exhaust received from the fuel cell system to form syngas containing at least 50 molar percent of a mixture of hydrogen and carbon monoxide, recycling a first portion of the syngas output from the exhaust processing system to the fuel cell system, and providing a second portion of the syngas output from the exhaust processing system to a syngas system to generate a product using the syngas.

## Description

### FIELD

Aspects of the present invention relate to fuel cell systems, and more specifically to integrated systems for generating and processing anode exhaust of a fuel cell system in order to generate syngas and then generate a product using the syngas.

### BACKGROUND

Fuel cells, such as solid oxide fuel cells, are electrochemical devices which can convert energy stored in fuels to electrical energy with high efficiencies. High temperature fuel cells include solid oxide and molten carbonate fuel cells. These fuel cells may operate using hydrogen and/or hydrocarbon fuels. There are classes of fuel cells, such as solid oxide regenerative fuel cells, that also allow reversed operation, such that oxidized fuel can be reduced back to unoxidized fuel using electrical energy as an input.

### SUMMARY

According to various embodiments, an integrated system includes a fuel cell system; an exhaust processing system configured to separate at least a portion of water and carbon dioxide from anode exhaust received from the fuel cell system to form syngas comprising at least 50 molar percent of a mixture of hydrogen and carbon monoxide; a processing conduit fluidly connecting the fuel cell system to the exhaust processing system, and configured to transfer the anode exhaust output from the fuel cell system to the exhaust processing system; a syngas system configured to generate a product using the syngas received from the exhaust processing system; a syngas recycle conduit fluidly connecting the exhaust processing system to the fuel cell system, and configured to recycle a first portion of the syngas output from the exhaust processing system to the fuel cell system; and a syngas conduit fluidly connecting the exhaust processing system to the syngas system, and configured to provide a second portion of the syngas output from the exhaust processing system to the syngas system.

According to various embodiments, a method includes providing fuel and air to a fuel cell system to generate electric power, anode exhaust and cathode exhaust, separating at least a portion of water and carbon dioxide from the anode exhaust received from the fuel cell system to form syngas containing at least 50 molar percent of a mixture of hydrogen and carbon monoxide, recycling a first portion of the syngas output from the exhaust processing system to the fuel cell system, and providing a second portion of the syngas output from the exhaust processing system to a syngas system to generate a product using the syngas.

### BRIEF DESCRIPTION OF THE DRAWINGS

The accompanying drawings, which are incorporated herein and constitute part of this specification, illustrate example embodiments of the invention, together with the general description given above and the detailed description given below.
FIG. 1 is a schematic representation of a fuel cell power module connected to an exhaust processing system, according to various embodiments of the present disclosure.
FIG. 2 is a schematic view of an integrated system including an exhaust processing system of FIG. 1, according to various embodiments of the present disclosure.
FIG. 3 is a schematic view of an integrated system including an alternative exhaust processing system, according to various embodiments of the present disclosure.
FIG. 4 is a schematic view of an integrated system including another alternative exhaust processing system, according to various embodiments of the present disclosure.

### DETAILED DESCRIPTION

The various embodiments will be described in detail with reference to the accompanying drawings. Wherever possible, the same reference numbers will be used throughout the drawings to refer to the same or like parts. References made to particular examples and implementations are for illustrative purposes and are not intended to limit the scope of the invention or the claims.

Solid oxide fuel cell (SOFC) systems may be operated using a hydrocarbon fuel, such as natural gas, methane, propane, etc., or a non-hydrocarbon fuel such as hydrogen (H₂) or ammonia. Anode exhaust generated by a SOFC system may include carbon dioxide and water, along with relatively small amounts of hydrogen, nitrogen (N₂), and carbon monoxide (CO). If the SOFC system is operated on ammonia fuel, then the anode exhaust may contain ammonia.

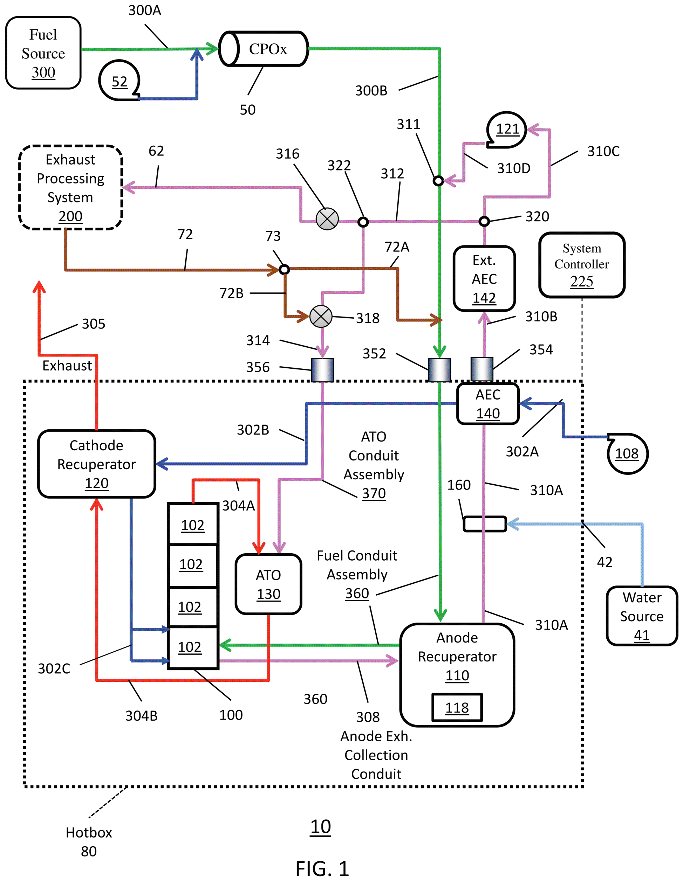

FIG. 1 contains a schematic representation of a fuel cell system (e.g., the SOFC system) power module 10, according to various embodiments of the present disclosure. Referring to FIG. 1, the power module 10 includes a hotbox 80 and various components disposed therein or adjacent thereto. The hotbox 80 may contain at least one fuel cell column 100 including one or more fuel cell stacks 102, such as one or more SOFC stacks containing alternating fuel cells and interconnects. One solid oxide fuel cell contains a ceramic electrolyte, such as yttria stabilized zirconia (YSZ), scandia stabilized zirconia (SSZ), scandia and ceria stabilized zirconia or scandia, yttria and ceria stabilized zirconia, an anode electrode, such as a nickel-YSZ, a nickel-SSZ or nickel-doped ceria cermet, and a cathode electrode, such as lanthanum strontium manganite (LSM). The interconnects may be metal alloy interconnects, such as chromium-iron alloy interconnects.

The hotbox 80 may also contain an anode recuperator heat exchanger 110, a cathode recuperator heat exchanger 120, an anode tail gas oxidizer (ATO) 130, an anode exhaust cooler heat exchanger 140, and an optional water injector 160. The power module 10 may also include a catalytic partial oxidation (CPOx) reactor 50, a CPOx blower 52 (e.g., a CPOx air blower), a system blower 108 (e.g., system air blower), and an anode recycle blower 121, which may be disposed outside of the hotbox 80. However, the present disclosure is not limited to any particular location for each of the components with respect to the hotbox 80.

The CPOx reactor 50 receives a fuel inlet stream from a fuel source 300 through a fuel conduit 300A. The fuel source 300 may be a fuel tank or a utility natural gas line including a valve to control an amount of fuel provided to the CPOx reactor 50. The CPOx blower 52 may provide air to the CPOx reactor 50 during system start-up. The fuel and/or air output from the CPOx reactor 50 may be provided by a fuel conduit 300B to fuel inlet 352 of a fuel conduit assembly 360. The fuel inlet 352 may be located in a wall of the hotbox 80. Fuel flows through the fuel conduit assembly 360 to the anode recuperator 110. The fuel is heated in the anode recuperator 110 by the anode exhaust (i.e., fuel exhaust of the fuel cell column 100) and the fuel then flows from the anode recuperator 110 to the fuel cell column 100 through the fuel conduit assembly 360.

The system blower 108 may be configured to provide an air stream (e.g., air inlet stream) to the anode exhaust cooler 140 through a first air conduit 302A. Air flows from the anode exhaust cooler 140 to the cathode recuperator 120 through a second air conduit 302B. The air is heated by the ATO 130 exhaust in the cathode recuperator 120. The air flows from the cathode recuperator 120 to the fuel cell column 100 through a third air conduit 302C.

An anode exhaust (e.g., fuel exhaust stream) generated in the fuel cell column 100 is provided to the anode recuperator 110 through anode exhaust collection conduit 308. The anode exhaust may contain unreacted fuel and may also be referred to herein as fuel exhaust. The anode exhaust may be recycled from the anode recuperator 110 to fuel conduit 300B by one or more recycling conduits, in order to mix the anode exhaust with incoming fresh fuel in the fuel conduit 300A. The one or more recycling conduits may include plural recycling conduits 310A-310D. First recycling conduit 310A may fluidly connect an outlet of the anode recuperator 110 to an inlet of the anode exhaust cooler 140. The second recycling conduit 310B may fluidly connect an outlet of the anode exhaust cooler 140 located in the hotbox 80 via an anode exhaust outlet 354 of the hotbox 80 to an inlet of an optional secondary anode exhaust cooler heat exchanger 142 located outside the hotbox 80. The third recycling conduit 310C may fluidly connect an outlet of the secondary anode exhaust cooler 142 to an inlet of an anode recycle blower 121. The fourth recycling conduit 310D may fluidly connect an outlet of the anode recycle blower 121 to the fuel conduit 300B at the mixer 311, where the anode exhaust mixes with the incoming fresh fuel.

The secondary anode exhaust cooler 142 may be, for example, a finned radiator or heat exchanger disposed outside of the hotbox 80. The secondary anode exhaust cooler 142 may be configured to cool the anode exhaust output from the hotbox 80 to protect the anode recycle blower 121 from thermal stress and/or damage. In some embodiments, the hotbox 80 may be disposed in a module cabinet (i.e., power module housing), and the secondary anode exhaust cooler 142 may be configured to cool the anode exhaust using ventilation air flowing through the module cabinet. One or more fans or blowers may be located in the module cabinet to provide the ventilation air.

Water flows from a water source, such as a water tank or a water pipe 41, to the water injector 160 through a water conduit 42. The water injector 160 may be configured to inject water into anode exhaust flowing through the first recycling conduit 310A. Heat from the anode exhaust vaporizes the water to generate steam which humidifies the anode exhaust. The humidified anode exhaust is then provided to the anode exhaust cooler 140. Heat from the anode exhaust provided to the anode exhaust cooler 140 may be transferred to the air inlet stream provided from the system blower 108 to the cathode recuperator 120. The cooled humidified anode exhaust may then be provided from the anode exhaust cooler 140 to the optional secondary anode exhaust cooler 142 via the second recycling conduit 310B. The anode exhaust may then be provided to the anode recycle blower 121 by the third recycling conduit 310C, before being provided to fuel conduit 300B by fourth recycling conduit 310D.

The power module 10 may also include one or more fuel reforming catalysts 118 located inside and/or downstream of the anode recuperator 110. The reforming catalyst(s) partially reform the humidified fuel mixture before it is provided to the fuel cell column 100.

Cathode (e.g., air) exhaust generated in the fuel cell column 100 is provided to the ATO 130 by a first cathode exhaust conduit 304A. The ATO exhaust flows from the ATO 130 to the cathode recuperator 120, through the second cathode exhaust conduit 304B. The ATO exhaust flows from the cathode recuperator 120 and out of the hotbox 80 through the cathode exhaust conduit 305.

The power module 10 may further include a system controller 225 configured to monitor and control various elements of the power module 10. The system controller 225 may include a central processing unit configured to execute stored instructions. For example, the system controller 225 may be configured to control fuel and/or air flow through the power module 10, according to fuel composition data.

Carbon dioxide may be separated from the anode exhaust and captured in an exhaust processing system 200, which may be located inside or outside the power module cabinet. For example, anode exhaust may contain about 54 mol % water, most of which can be removed using a condenser. A remaining dry content of anode exhaust may include about 62 mol % CO₂, 25 mol % H₂, 12 mol % CO and 1 mol % N₂.

In prior SOFC systems, anode exhaust may be routed directly to the ATO using an ATO injector and/or conduits disposed within the system hotbox 80. However, when the anode exhaust is routed outside of the hotbox 80, such as when anode exhaust is provided to an exhaust processing system 200, conduits used to return the anode exhaust to the ATO 130 may significantly restrict the flow of the anode exhaust. In particular, the present inventors determined that space constraints within a hotbox may limit the size of such conduits, resulting in a significant system pressure drop.

Accordingly, the power module 10 may include a bypass conduit 312, a return conduit 314, a processing conduit 62, a bypass valve 316, a return valve 318 (e.g., ATO valve), a first splitter 320, and a second splitter 322. The bypass conduit 312 and the processing conduit 62 may fluidly connect the third recycling conduit 310C to the exhaust processing system 200. The inlet of the bypass conduit 312 may be fluidly connected to the third recycling conduit 310C at the first splitter 320. The outlet of the bypass conduit 312 may be fluidly connected to the inlets of the processing conduit 62 and the return conduit 314 at the second splitter 322.

The return conduit 314 may fluidly connect the bypass conduit 312 to the anode exhaust inlet 356 of the hotbox 80. The ATO conduit assembly 370 may fluidly connect the anode exhaust inlet 356 to the ATO 130. The bypass valve 316 is located on the processing conduit 62 and is configured to control anode exhaust flow through the processing conduit 62. The return valve 318 is located on the return conduit 314 and is configured to control the anode exhaust flow through the return conduit 314. The valves 316, 318 may be electrically operated valves, such as solenoid valves or the like. Alternatively, the two valves 316, 318 and the second splitter 322 may be replaced by a single three-way valve which controls the relative amounts of anode exhaust flowing through the processing conduit 62 and the return conduit 314.

In one embodiment, a syngas recycle conduit 72 fluidly connects the exhaust processing system 200 to the power module 10. Specifically, the exhaust processing system 200 may be configured to separate at least a portion of water and carbon dioxide (and optionally) hydrogen from the anode exhaust, as will be described in more detail below. The remaining anode exhaust comprises a syngas. The syngas recycle conduit 72 is configured to recycle at least a first portion of the syngas from the exhaust processing system 200 back to the power modules 10 of the fuel cell system. In one embodiment, the syngas recycle conduit 72 includes an optional splitter 73 which splits the syngas recycle conduit 72 into a first branch 72A and an optional second branch 72B. The first branch 72A is fluidly connected to the fuel conduit 300B, and is configured to recycle a first part of the first portion of the syngas from the exhaust processing system 200 into the fuel inlet stream provided to the stacks 102 of the fuel cell column 100. The second branch 72B is fluidly connected to the return conduit 314, and is configured to recycle a second part of the first portion of the syngas from the exhaust processing system 200 into the ATO 130. Specifically, since the syngas may contain nitrogen, oxidizing and venting the second part of the first portion of the syngas prevents nitrogen buildup in the recycled fuel loop. If the optional splitter 73 and the second branch 72B are omitted, nitrogen buildup may be mitigated by providing the anode exhaust from at least one power module 10 to the ATO 130 of that power module 10 instead of to the exhaust processing system 200, while the anode exhaust from at least one other power module 10 is provided to the exhaust processing system 200.

In the embodiment shown in FIG. 1, the bypass conduit 312 is fluidly connected to the third recycling conduit 310C upstream of the recycle blower 121, with respect to an anode exhaust flow direction through the third recycling conduit 310C. In an alternative embodiment, the bypass conduit 312 may be fluidly connected to the fourth recycling conduit 310D, downstream of the anode recycle blower 121, in order to provide additional anode exhaust flow pressure through the bypass conduit 312.

Locating the bypass valve 316 and the return valve 318 outside of the hotbox 80 may provide the benefit of protecting the valves 316, 318 from damage due to exposure to high temperatures inside of the hotbox 80. For example, this configuration allows for the use of relatively inexpensive valves, as compared to valves rated for high temperature operation.

The system controller 225 may be configured to control the operation of the valves 316, 318 and/or the recycle blower 121 to control anode exhaust flow to the exhaust processing system 200, the ATO 130, and/or the anode recuperator 110.

For example, during system startup, shutdown, and/or transient operation, the bypass valve 316 may be closed, the return valve 318 may be opened, such that anode exhaust is provided to the ATO 130 generate heat and bring the power module 10 up to the system steady-state operating temperature (e.g., a temperature above 700 °C, such as from 750 °C to 900 °C). During the steady-state operation, the bypass valve 316 may be opened and the return valve 318 may be closed, such that all or a majority of the anode exhaust not required for anode recycle is provided to the exhaust processing system 200. In some embodiments, the return valve 318 may be partially opened during steady-state operation, in order to provide the ATO 130 with a relatively reduced amount of anode exhaust to provide additional heat to the system.

In some embodiments, an amount of the anode exhaust that is recycled to the fuel cell column 100 through the fourth recycling conduit 310D, may be at least partially controlled by controlling the speed of the recycle blower 121. Thus, the remaining amount of the anode exhaust that is provided to the exhaust processing system 200 and/or the ATO 130 through the bypass conduit 312 may also be controlled.

The exhaust processing system 200 may be configured to separate carbon dioxide from the anode exhaust in order to generate a commercially valuable carbon dioxide product. The exhaust processing system 200 may also be configured to remove water from the anode exhaust. The exhaust processing system 200 may also include electrochemical pumps and/or distillation systems, to separate other valuable exhaust components, such as hydrogen and/or carbon monoxide. In some embodiments, the exhaust processing system 200 may be configured to receive anode exhaust from multiple hotboxes 80 (e.g., exhaust processing system 200 may be connected to multiple power modules 10).

In some embodiments, the amount of H₂ included in the anode exhaust may be increased by lowering the fuel utilization efficiency of the power module 10 below the baseload configuration (e.g., where the power module 10 is configured to operate to meet the electrical power load demand). However, lower fuel utilization efficiencies may lead to a higher output current provided to the load of the power module 10 in order to operate the power module in a thermally stable condition without any fuel being provided to the ATO 130. Lowering the fuel utilization rate may also decrease the AC power efficiency of the power module 10. However, the present inventors have determined that such operation may still be economically advantageous, since a hydrogen (H₂) product is nearly always more valuable than the amount of electricity that could be generated by recycling the H₂ to the power module 10.

The power module 10 may be operated at a fuel cell column fuel utilization rate that is lower than the thermal stability point of the fuel cell column, in order to increase the H₂ content of the anode exhaust. In other words, due to the relatively low fuel utilization rate, the fuel cell columns 100 may generate less heat than required to maintain the fuel cell columns 100 at a desired steady-state operating temperature. For example, the fuel cell columns 100 may have a fuel utilization rate of less than the baseload fuel utilization rate, such as a fuel utilization rate below 85%, such as a fuel utilization rate ranging from about 65% to about 85%, such as from about 70% to about 80%.

In some embodiments, the power module 10 may operate in a start-up mode, and then after reaching a steady-state mode temperature range, the power module 10 may operate in the steady-state mode by repeatedly cycling between an anode exhaust export mode and a thermal recovery mode, during which the temperature of the fuel cell columns 100 may fluctuate while remaining within a suitable operating temperature range. For example, during the anode exhaust export mode, all of the anode exhaust may be exported from the power module 10 and provided to the exhaust processing system 200, by opening the bypass valve 316 and closing the ATO valve 318. In other words, no anode exhaust is provided to the ATO 130 during the anode exhaust export mode and the amount of exported anode exhaust is maximized. During the anode exhaust export mode, the temperature of the fuel cell columns 100 may drop due to the lower fuel utilization rate of the fuel cell columns 100. For example, in some embodiments, the temperature of the fuel cell columns 100 may be reduced from a first temperature to a lower second temperature during the anode exhaust export mode.

In some embodiments, the first and second temperatures may be a set maximum fuel cell column operating temperature and a set minimum fuel cell column operating temperature, which may be selected by a system user. The range between the first and second temperatures may be referred to as a "dead band" power module operating temperature range. For example, the fuel cell columns 100 may operate with acceptable efficiency and fuel utilization over a range of operating temperatures. For example, upper and lower limits of a dead band operating temperature range may vary by 20 °C, by 15 °C, or by 10 °C. In some embodiments, the first temperature may range from about 815 °C to about 825 °C, such as about 820 °C, and the second temperature may range from about 805 °C to about 815 °C, such as about 810 °C.

Once the column 100 reaches the second temperature, the power module 10 may begin operating in the thermal recovery mode. In particular, the anode exhaust may be provided to the ATO 130 by completely or partially closing the bypass valve 316 and opening the ATO valve 318. The anode exhaust is oxidized in the ATO 130 by the cathode exhaust to heat the fuel cell columns 100. The thermal recovery mode may continue until the fuel cell columns 100 return to the first temperature. The power module 10 may then resume operating in the anode exhaust export mode, and the cycle may repeat.

In some embodiments, the system controller 225 may be configured to monitor the temperature of the fuel cell columns 100 using one or more thermocouples or other temperature detectors, in order to determine whether the power module 10 should be operated in the anode exhaust export mode or the thermal recovery mode. In other embodiments, the controller 225 may be configured to periodically switch between the exhaust export and thermal recovery modes. For example, the power module 10 may be operated in the thermal recovery mode for less than 60 seconds (e.g., 5 seconds) every minute, or for less than 10 minutes (e.g., 1 minute) every 10 minutes, in order to maintain the fuel cell columns 100 at a temperature within the dead band operating temperature range.

In embodiments where multiple power modules 10 are connected to the exhaust processing system 200, a substantially uniform amount of anode exhaust may be supplied to the exhaust processing system 200 by operating a consistent number of the power modules 10 in the exhaust export mode. For example, a first power module 10 may be operated in thermal recovery mode, while a remainder of the power modules 10 operate in exhaust export mode. Then a second power module 10 may be operated in thermal recovery mode, while the remainder of the power modules 10 operate in export mode. The process can continue for each power module 10.

Diverting a time dependent portion of the fuel to the ATO 130 may also permit the fuel cell columns 100 to operate at lower currents, which also provides the ability to respond to planned loads as a function of time. This means that the power module 10 can be programmed to run at different power levels at different times of day, at different days of week, during different daily weather forecasts (assuming power required is a function of ambient weather), during different seasons, and/or a combination of one or more of the above. This also allows for a reduction in AC power generation (e.g., from 100 % to 60 %) by the power modules 10. As a result, various embodiments provide plural (e.g., two) customer specific set points as a function of time to best meet the customers demand by foregoing the traditional practice of optimizing efficiency and fuel utilization for constant baseload electricity production.

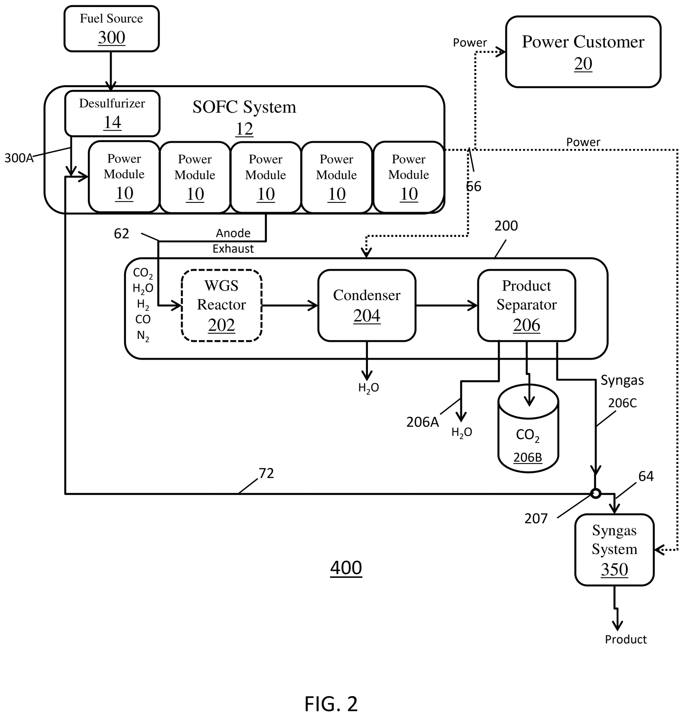

FIG. 2 is a schematic view of an integrated system 400, according to various embodiments of the present disclosure. Referring to FIGS. 1 and 2, the integrated system 400 may include a fuel cell system (such as the SOFC system) 12, which includes the above described the power modules 10 and the exhaust processing system 200 as shown in FIG. 1, and also includes a syngas system 350. The exhaust processing system 200 may include an optional water gas shift (WGS) reactor 202, a condenser 204, and a product separator 206 that are configured to process the anode exhaust stream received from the SOFC system 12 comprising one or more power modules 10. In an alternative embodiment, the WGS reactor 202 may be omitted. The exhaust processing system 200 or parts thereof may be located in the SOFC system 12 or outside the SOFC system 12. For example, the WGS reactor 202 may be located in one or more power modules 10 of the SOFC system 12, in a separate module of the SOFC system 12 different from the power modules 10, or separately from the SOFC system 12.

According to various embodiments, anode exhaust provided from SOFC power modules 10 may include water, carbon dioxide, hydrogen, carbon monoxide, nitrogen and any residual hydrocarbon fuel. The anode exhaust may be provided to the optional WGS reactor 202, where CO and water may be reacted to generate additional hydrogen and carbon dioxide in a water-gas shift reaction. Alternatively, the WGS reactor 202 may be omitted. In an alternative embodiment where more syngas product and less carbon dioxide product is desired, the WGS reactor 202 may comprise a catalytic reverse water gas shift reactor in which hydrogen and carbon dioxide are converted to water and carbon monoxide. The reverse water gas shift reactor produces additional carbon monoxide which may be mixed with remaining hydrogen in the anode exhaust to form additional syngas product.

The anode exhaust may then be provided to the condenser 204, which may be configured to reduce the water content of the anode exhaust, by condensing liquid water out of the anode exhaust stream. The anode exhaust exiting the condenser 204 may optionally be dried in a dryer (e.g., an absorption dryer (e.g., glycol based dryer) or a temperature swing adsorption bed dryer).

The anode exhaust may then be provided to the product separator 206. The product separator 206 may be, for example, a cryogenic device, a pressure swing adsorption device, a chemical absorption device (e.g. an alkanol amine or a mixture of dimethyl ethers of polyethylene glycol (for example Selexol^{®}) based absorption device), an electrochemical device (e.g., an electrochemical separation membrane), or a distillation device configured to separate the anode exhaust into a water discharge stream that is output via a water discharge conduit 206A, a CO₂ product stored in a carbon dioxide storage vessel 206B, and a remaining syngas stream output via a syngas discharge conduit 206C. Thus, the product separator 206 may comprise at least a carbon dioxide separator and optionally a water separator in addition to the carbon dioxide separator.

The syngas discharge conduit 206C may terminate in a splitter 207, which splits conduit 206C into a syngas conduit 64 and the above described syngas recycle conduit 72. The syngas recycle conduit 72 recycles the first portion of the syngas back to the fuel cell system 12 (e.g., to the power modules 10 as described above). The syngas conduit 64 provides a second portion of the syngas to the syngas system 350. The syngas system 350 utilizes the syngas to generate a product, as discussed in detail below. The syngas may include mostly a mixture of hydrogen and carbon monoxide, with some remaining nitrogen and carbon dioxide. For example, the syngas may include at least 50 molar percent, such as 55 to 95 molar percent of a mixture of hydrogen and carbon monoxide, and less than 50 molar percent such as 5 to 45 molar percent nitrogen and carbon dioxide. In one embodiment, the syngas may include 40 to 60 molar percent hydrogen, 15 to 35 molar percent carbon monoxide, 4 to 25 molar percent nitrogen, 1 to 20 molar percent carbon monoxide, and less than 1 molar percent hydrocarbon fuel. The syngas may be recycled from the exhaust processing system 200 to the power module 10 via the syngas recycle conduit 72 during normal operation or when the syngas system 350 is unavailable (e.g., for maintenance).

In various embodiments, the SOFC system 12 may be electrically connected to the exhaust processor 200, the syngas system 350, and/or a power customer 20 by a local power grid 66. Power generated by the SOFC system 12 may be used to power components of the exhaust processor 200 and/or the syngas system 350. As such, the power modules 10 may operate as a low carbon power source for the exhaust processor 200, the syngas system 350 and/or a power customer 20 (e.g., any additional load) electrically connected to the local power grid 66. In some embodiments, the local power grid 66 may optionally connect the SOFC system 12 to the power customer 20, such as a data center, a residential building or a commercial building.

Referring to FIGS. 1 and 2, in one embodiment, the fuel cell system 12 comprises a first power module 10 including a first set of stacks 102 of solid oxide fuel cells, a first anode tail gas oxidizer (ATO) 130, and a first exhaust conduit 350 which is fluidly connected to the first ATO 130 and is configured to remove ATO exhaust generated by the first ATO 130 from the first power module 10 (e.g., through the cathode recuperator 120). The syngas recycle 72 conduit includes a splitter 73, a first branch 72A fluidly connecting the splitter 73 to the first set of stacks 102 and configured to provide a first part of the first portion of the anode exhaust to the first stacks 102, and a second branch 72B fluidly connecting the splitter 73 to the first ATO 130 and configured to provide a second part of the first portion of the anode exhaust to the first ATO 130.

In one embodiment shown in FIG. 2, the fuel cell system 12 may also include a desulfurizer 14. The desulfurizer 14 may comprise a system level or a site level desulfurizer module which contains one or more desulfurization beds which contain a material which absorbs and/or adsorbs sulfur species (e.g., H₂S, etc.) present in the fuel (e.g., natural gas) provided from the fuel source 300. The desulfurizer 14 removes all or a majority of the sulfur species form the fuel inlet stream that is then provided from the desulfurizer 14 to the power modules 10 via the fuel conduit 300A. The system controller 225 may receive data from various flow rate and fuel sensors that corresponds to the flow rate of the fuel inlet stream (e.g., natural gas) output from the desulfurizer 14, the measured, stored or assumed composition of the fuel inlet stream (e.g., natural gas), the flow rate of the syngas recycle stream in the syngas recycle conduit 72, and the measured composition of the syngas recycle stream in the syngas recycle conduit 72. The system controller 225 may then control the operation of the power modules 10 (e.g., by controlling the flow of the fuel inlet stream and/or the syngas recycle stream, the speed of the blower(s) 108 and/or 121, and/or the amount of electric power output by the power modules 10).

In one embodiment, the fuel cell system 12 further comprises a plurality of additional power modules 10, as shown in FIG. 2. Each of the additional power modules 10 includes additional set of stacks 102 of solid oxide fuel cells, an additional ATO 130, and an additional exhaust conduit 305 which is fluidly connected to the respective additional ATO 130 and is configured to remove ATO exhaust from the respective additional power module 10. The first branch 72A fluidly further connects the splitter 73 to the additional sets of stacks 102 and is further configured to provide the first part of the first portion of the anode exhaust to the additional stacks 102. The second branch 72B further fluidly connects the splitter 73 to the additional ATOs 130 and is further configured to provide the second part of the first portion of the anode exhaust to the additional ATOs 130.

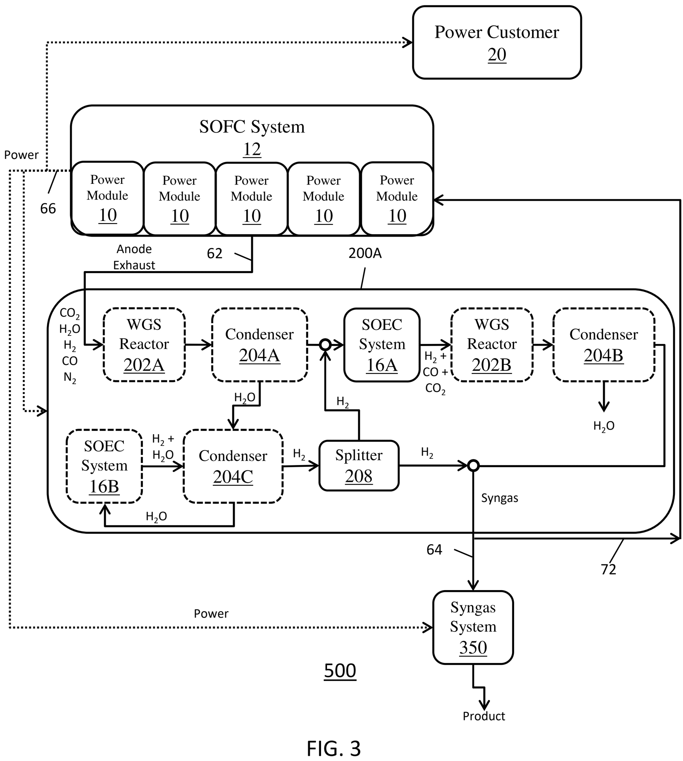

FIG. 3 is a schematic view of an integrated system 500 including an alternative exhaust processing system 200A, according to various embodiments of the present disclosure. The integrated system 500 may be similar to the integrated system 400. Accordingly, only the differences therebetween will be discussed in detail.

Referring to FIGS. 1 and 3, the integrated system 500 may include the fuel cell system (e.g., the SOFC system 12), an exhaust processing system 200A, and a syngas system 350. The exhaust processing system 200A may be configured to receive the anode exhaust from power modules 10 of the fuel cell system 12 via the processing conduit 62 to provide a first portion of the syngas to the fuel cell system 12 via the syngas recycle conduit 72 and to provide a second portion of the syngas to the syngas system 350 via the syngas conduit 64.

For example, the SOFC system 12 may be configured to meet a significant power demand customer (e.g., a data center or a utility scale power plant) that can be collocated with the syngas system (e.g., a syngas consuming system) 350, such as a green liquid fuel generation process or any other significant syngas user. Rather than purifying the CO₂ from the anode exhaust product and feeding it to a downstream syngas consuming process, the exhaust processing system 200A may be configured to directly generate syngas from anode exhaust of the power modules 10. The syngas that may be further processed into a desired product.

The exhaust processing system 200A may be configured to adjust the composition of the anode exhaust, based on the requirements of a downstream syngas system 350. In particular, the exhaust processing system 200A may include various components based on the composition of the anode exhaust received from the SOFC system 12. For example, if the anode exhaust has too much fully oxidized content (CO₂ and H₂O instead of CO and H₂), the exhaust processing system 200A may include an electrolyzer system (such as a solid oxide electrolyzer cell (SOEC) system) 16A to convert the CO₂ and H₂O to CO and H₂, thus raising the concentration of the syngas components. The SOEC system 16A may be operated using renewable green power (e.g., power from the fuel cell system 12, solar power, wind power, etc.).

The exhaust processing system 200A may also include an optional WGS reactor 202A and an optional condenser 204A upstream of the SOEC system 16A, with respect to anode exhaust flow direction. In an alternative embodiment, the exhaust processing system 200A may include an optional WGS reactor 202B and an optional condenser 204B downstream of the SOEC system 16A in addition to or instead of the upstream WGS reactor 202A and the upstream condenser 204A. The WGS reactors 202A, 202B may be operated to react the H₂O and CO in the anode exhaust to form additional H₂ and CO₂.

In some embodiments, the exhaust processing system 200A may also include an optional additional SOEC system 16B and condenser 204C configured to provide H₂ to the anode exhaust, upstream and/or downstream of the SOEC system 16A. In particular, the SOEC system 16B and the condenser 204C may be configured to raise the H₂:CO ratio of the anode exhaust provided to the syngas system 350. If the product syngas allows a high enough CO₂ + H₂O content, the SOEC systems 16A and/or 16B may be operated without steam recycle in single pass mode. The exhaust processing system 200A may include a splitter 208 to control where the hydrogen generated by the SOEC system 16B is provided to the anode exhaust stream. For example, the splitter 208 may be configured to provide between 0 and 100% of the H₂ generated by the SOEC system 16B upstream of the SOEC system 16A and a remaining amount of the H₂ generated by the SOEC system 16B downstream of the SOEC system 16A.

In some embodiments, hot anode exhaust (having a temperature of 150 °C - 400 °C) can be output from the power modules 10 (depending on requirements) by pulling out some or all of the anode exhaust before it enters the anode exhaust cooler 140 and or before it enters the supplemental anode exhaust cooler 142. This hot anode exhaust may provide multiple benefits, such as allowing for the use of a low temperature WGS reactor to maximize H₂ content. This hot anode exhaust may also have a higher water content, which may increase the H₂ content of the anode exhaust after passing through the SOEC system 16A, and/or may provide a higher temperature feed to the SOEC system 16A, if it does not need to be controlled by a mass flow controller (MFC) based on temperature limitations of an MFC.

The condensers 204A, 204B, if present, may cool the anode exhaust. Water output from the condensers 204A, 204B may be available for use by standard collocated SOEC's or used on site for cooling tower makeup water if the site has a cooling tower.

Accordingly, the exhaust processing system 200A may provide residual CO and H₂ in the anode exhaust product for use in the downstream syngas system 350. Purification of CO₂ for sequestration or H₂ for sale is no longer required, saving both capital cost and operating parasitic power.

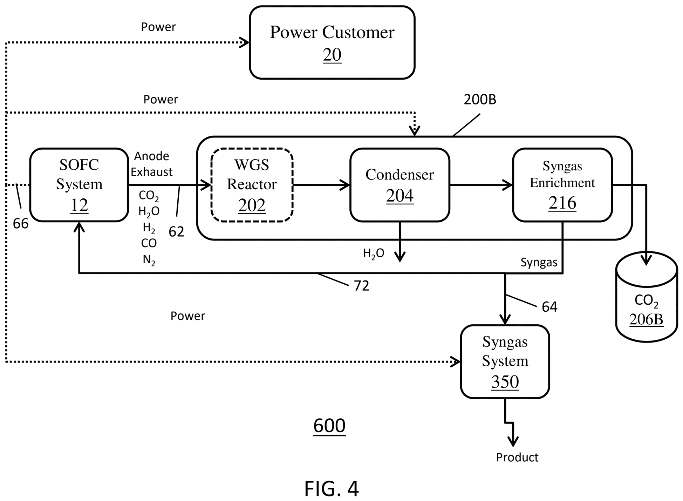

FIG. 4 is a schematic view of an integrated system 600 including an alternative exhaust processing system 200B, according to various embodiments of the present disclosure. The integrated system 600 may be similar to the integrated system 400. Accordingly, only the differences therebetween will be discussed in detail.

Referring to FIGS. 1 and 4, the integrated system 600 may include the fuel cell system (e.g., the SOFC system) 12, the syngas system 350, and an exhaust processing system 200B. The fuel cell system 12 may be electrically connected to the exhaust processing system 200B, the syngas system 350, and/or a power customer 20 by the local power grid 66.

The exhaust processing system 200B may be configured to generate purified CO₂ and syngas using anode exhaust received from one or more power modules 10 in SOFC system 12. In particular, the exhaust processing system 200B may include an optional WGS reactor 202, the condenser 204, and a syngas enrichment system 216. The WGS reactor 202 may be used to increase the H₂ and CO₂ content of the anode exhaust. The anode exhaust may be partially or completely dehydrated.

The anode exhaust may then be provided to the syngas enrichment system 216 configured to generate syngas of a desired H₂ and CO content. For example, the enrichment system 216 may include a cryogenic process, a pressure swing adsorption process, a membrane process, an amine-based absorber/stripper process, or the like configured to adjust the composition of the anode exhaust. For example, all or a portion of the CO₂ component of the anode exhaust may be separated from the syngas component and stored in storage vessel 206B. Thus, in one embodiment, the syngas enrichment system 216 may comprise at least a carbon dioxide separator. In addition, the syngas enrichment system 216 may be configured to adjust the H₂ to CO ratio of the anode exhaust to match the requirements of the syngas system 350. The amount of CO, H₂, H₂O, N₂, and hydrocarbons that are permitted to remain in the purified CO₂ product depends on the intended use of the CO₂ (e.g., on the purity of the carbon dioxide desired for the intended use of the purified carbon dioxide).

The final syngas product may be provided via the syngas conduit 64 to the syngas system 350. H₂ from the above described electrolyzer system(s) may be added to increase the H₂/CO ratio of the final syngas fed to the syngas system 350.

Referring to FIGS. 2-4, the syngas system 350 may comprise any system, facility, factory, apparatus, method, etc., that uses the syngas to generate a higher value product. For example, the syngas system 350 may comprise facilities that use syngas to generate products such as liquid hydrocarbons, ammonia, methanol, or the like. The syngas system 350 may be physically integrated with one, plural or all components of the exhaust processing system (200, 200A or 200B). For example, the WGS reactor 202 may comprise a portion of the syngas system 350. Alternatively, the syngas system 350 may be physically separate from the exhaust processing system (200, 200A or 200B).

In one embodiment, the syngas system 350 may include a catalytic reactor configured to perform a steam reformation process, an autothermal reformation process, or a partial oxidation process to convert syngas received from the exhaust processor into purified hydrogen (H₂) product. In some embodiments, this process may include carbon capture and may be referred to as a blue hydrogen process. The syngas produced by the embodiment exhaust processing system (200, 200A or 200B) may be used as the sole source of syngas or as a supplemental source of syngas. The generated hydrogen gas may be supplied to a subsequent process to generate more complex chemicals. For example, the syngas system 350 may include a Haber reactor configured to convert hydrogen and atmospheric nitrogen (N₂) into ammonia product using the Haber process.

In another embodiment, the syngas system 350 may include an ethanol reactor containing a catalyst (e.g., Rh-Mn or Cu-Co) which directly catalytically converts the syngas to ethanol in a one step process or a multi-step process which includes one or more intermediate steps. Alternatively, the syngas system 350 may comprise a system which generates a liquid fuel, such as ethanol, from the syngas, in a multi-step process.

In another embodiment, the syngas system 350 may include a methanol reactor configured to convert syngas into methanol. Optionally, the syngas system 350 may utilize the methanol to produce more complex compounds, such as ethylene, acetic acid, formaldehyde, dimethyl ether, methyl acetate, polyolefins, or the like. For example, the syngas system 350 may include a catalytic reactor containing a catalyst (e.g., Rh-Mn or Cu-Co) which indirectly converts the syngas via methanol or dimethyl ether (DME) synthesis to an intermediate product, followed by carbonylation with carbon monoxide and subsequent hydrogenation of acetic acid or methyl acetate, which may occur in a separate reactor or in the same reactor.

In another embodiment, syngas system 350 may be a fuel production system that includes a Fischer-Tropsch reactor that utilizes the Fischer-Tropsch process to convert syngas into liquid hydrocarbons, such as aviation fuel, diesel, gasoline, naptha, lubricants, or the like.

In another embodiment, the syngas system 350 may include an iron reduction reactor that utilizes syngas to reduce iron ore to generate a sponge iron product. For example, the syngas may be used as a reducing agent to reduce iron oxide to sponge iron.

Accordingly, the embodiments of the present disclosure provide integrated systems that synergistically integrate a fuel cell system (e.g., a SOFC system), an exhaust processing system, and a syngas system, to reduce overall system carbon emissions and/or improve overall system efficiency.

The preceding description of the disclosed aspects is provided to enable any person skilled in the art to make or use the present invention. Various modifications to these aspects will be readily apparent to those skilled in the art, and the generic principles defined herein may be applied to other aspects without departing from the scope of the invention. Thus, the present invention is not intended to be limited to the aspects shown herein but is to be accorded the widest scope consistent with the principles and novel features disclosed herein.
The invention also refers to the following embodiments, wherein the term "claim" means "embodiment":
1. An integrated system, comprising:
   a fuel cell system;
   an exhaust processing system configured to separate at least a portion of water and carbon dioxide from anode exhaust received from the fuel cell system to form syngas comprising at least 50 molar percent of a mixture of hydrogen and carbon monoxide;
   a processing conduit fluidly connecting the fuel cell system to the exhaust processing system, and configured to transfer the anode exhaust output from the fuel cell system to the exhaust processing system;
   a syngas system configured to generate a product using the syngas received from the exhaust processing system;
   a syngas recycle conduit fluidly connecting the exhaust processing system to the fuel cell system, and configured to recycle a first portion of the syngas output from the exhaust processing system to the fuel cell system; and
   a syngas conduit fluidly connecting the exhaust processing system to the syngas system, and configured to provide a second portion of the syngas output from the exhaust processing system to the syngas system.
2. The integrated system of claim 1, wherein the syngas system comprises an iron ore reduction reactor configured to use the syngas to reduce iron ore and generate the product comprising sponge iron.
3. The integrated system of claim 1, wherein the syngas system comprises a catalytic reactor configured to perform a steam reformation process, an autothermal reformation process, or partial oxidation process to convert the syngas into the product comprising purified hydrogen.
4. The integrated system of claim 3, wherein the syngas system further comprises a Haber reactor configured to convert the purified hydrogen and atmospheric nitrogen into ammonia using the Haber process.
5. The integrated system of claim 1, wherein the syngas system comprises a Fischer-Tropsch reactor configured to convert the syngas into the product comprising liquid hydrocarbons using the Fischer-Tropsch process.
6. The integrated system of claim 1, wherein the syngas system comprises a methanol reactor configured to convert the syngas into the product comprising methanol.
7. The integrated system of claim 1, further comprising a local power grid configured to electrically connect an electrical output of the fuel cell system to electrical inputs of an electrical load, the exhaust processing system and the syngas system.
8. The integrated system of claim 1, wherein the exhaust processing system comprises:
   a condenser configured to remove water from the anode exhaust; and
   a carbon dioxide separator configured to separate carbon dioxide from the syngas in the anode exhaust received from the condenser.
9. The integrated system of claim 1, wherein:
   the fuel cell system comprises a first powder module including a first set of stacks of solid oxide fuel cells, a first anode tail gas oxidizer (ATO), and a first exhaust conduit which is fluidly connected to the first ATO and is configured to remove ATO exhaust from the first power module; and
   the syngas recycle conduit includes a splitter, a first branch fluidly connecting the splitter to the first set of stack, and configured to provide a first part of the first portion of the anode exhaust to the first stacks, and a second branch fluidly connecting the splitter to the first ATO, and configured to provide a second part of the first portion of the anode exhaust to the first ATO.
10. The integrated system of claim 9, wherein:
   the fuel cell system further comprises a plurality of additional power modules;
   each of the additional power modules includes additional set of stacks of solid oxide fuel cells, an additional anode tail gas oxidizer (ATO), and an additional exhaust conduit which is fluidly connected to the respective additional ATO and is configured to remove ATO exhaust from the respective additional power module;
   the first branch fluidly further connects the splitter to the additional sets of stacks and is further configured to provide the first part of the first portion of the anode exhaust to the additional stacks; and
   the second branch further fluidly connects the splitter to the additional ATOs and is further configured to provide the second part of the first portion of the anode exhaust to the additional ATOs.
11. A method, comprising:
   providing fuel and air to a fuel cell system to generate electric power, an anode exhaust and a cathode exhaust;
   separating at least a portion of water and carbon dioxide from the anode exhaust received from the fuel cell system to form syngas comprising at least 50 molar percent of a mixture of hydrogen and carbon monoxide;
   recycling a first portion of the syngas output from the exhaust processing system to the fuel cell system; and
   providing a second portion of the syngas output from the exhaust processing system to a syngas system to generate a product using the syngas.
12. The method of claim 11, wherein the syngas system comprises an iron ore reduction reactor which uses the syngas to reduce iron ore and generate the product comprising sponge iron.
13. The method of claim 11, wherein the syngas system comprises a catalytic reactor which performs perform a steam reformation process, an autothermal reformation process, or partial oxidation process to convert the syngas into the product comprising purified hydrogen.
14. The method of claim 13, wherein the syngas system further comprises a Haber reactor which converts the purified hydrogen and atmospheric nitrogen into ammonia using the Haber process.
15. The method of claim 11, wherein the syngas system comprises a Fischer-Tropsch reactor which converts the syngas into the product comprising liquid hydrocarbons using the Fischer-Tropsch process.
16. The method of claim 11, wherein the syngas system comprises a methanol reactor which converts syngas into the product comprising methanol.
17. The method of claim 11, further comprising providing the electric power from the fuel cell system to the syngas system, an electrical load, and an exhaust processing system which separates the water and the carbon dioxide from the anode exhaust.
18. The method of claim 11, wherein the step of separating the water and the carbon dioxide from the anode exhaust comprises:
   condensing water from the anode exhaust; and
   separating the carbon dioxide from the syngas in the anode exhaust after the condensing the water from the anode exhaust.
19. The method of claim 11, wherein:
   the fuel cell system comprises a first powder module including a first set of stacks of solid oxide fuel cells, a first anode tail gas oxidizer (ATO), and a first exhaust conduit which is fluidly connected to the first ATO and removes ATO exhaust from the first power module;
   a first part of the first portion of the anode exhaust is provided to the first stacks, and a second part of the first portion of the anode exhaust is provided to the first ATO;
   the fuel cell system further comprises a plurality of additional power modules;
   each of the additional power modules includes additional set of stacks of solid oxide fuel cells, an additional anode tail gas oxidizer (ATO), and an additional exhaust conduit which is fluidly connected to the respective additional ATO and is configured to remove ATO exhaust from the respective additional power module;
   the first part of the first portion of the anode exhaust is further provided to the additional stacks; and
   the second part of the first portion of the anode exhaust is further provided to the additional ATOs.
20. The method of claim 11, further comprising controlling the fuel cell system based on a flow rate and a composition of the fuel, and based on a flow rate and a composition of the first portion of the syngas.

## Claims

1. An integrated system, comprising:
a fuel cell system;
an exhaust processing system configured to separate at least a portion of water and carbon dioxide from anode exhaust received from the fuel cell system to form syngas comprising at least 50 molar percent of a mixture of hydrogen and carbon monoxide;
a processing conduit fluidly connecting the fuel cell system to the exhaust processing system, and configured to transfer the anode exhaust output from the fuel cell system to the exhaust processing system;
a syngas system configured to generate a product using the syngas received from the exhaust processing system;
a syngas recycle conduit fluidly connecting the exhaust processing system to the fuel cell system, and configured to recycle a first portion of the syngas output from the exhaust processing system to the fuel cell system; and
a syngas conduit fluidly connecting the exhaust processing system to the syngas system, and configured to provide a second portion of the syngas output from the exhaust processing system to the syngas system.

2. The integrated system of claim 1, wherein the syngas system comprises an iron ore reduction reactor configured to use the syngas to reduce iron ore and generate the product comprising sponge iron.

3. The integrated system of claim 1, wherein:
the syngas system comprises a catalytic reactor configured to perform a steam reformation process, an autothermal reformation process, or partial oxidation process to convert the syngas into the product comprising purified hydrogen; and
the syngas system further comprises a Haber reactor configured to convert the purified hydrogen and atmospheric nitrogen into ammonia using the Haber process.

4. The integrated system of claim 1, wherein the syngas system comprises a Fischer-Tropsch reactor configured to convert the syngas into the product comprising liquid hydrocarbons using the Fischer-Tropsch process.

5. The integrated system of claim 1, wherein the syngas system comprises a methanol reactor configured to convert the syngas into the product comprising methanol.

6. The integrated system of claim 1, further comprising a local power grid configured to electrically connect an electrical output of the fuel cell system to electrical inputs of an electrical load, the exhaust processing system and the syngas system.

7. The integrated system of claim 1, wherein the exhaust processing system comprises:
a condenser configured to remove water from the anode exhaust; and
a carbon dioxide separator configured to separate carbon dioxide from the syngas in the anode exhaust received from the condenser.

8. The integrated system of claim 1, wherein:
the fuel cell system comprises a first powder module including a first set of stacks of solid oxide fuel cells, a first anode tail gas oxidizer (ATO), and a first exhaust conduit which is fluidly connected to the first ATO and is configured to remove ATO exhaust from the first power module;
the syngas recycle conduit includes a splitter, a first branch fluidly connecting the splitter to the first set of stack, and configured to provide a first part of the first portion of the anode exhaust to the first stacks, and a second branch fluidly connecting the splitter to the first ATO, and configured to provide a second part of the first portion of the anode exhaust to the first ATO;
the fuel cell system further comprises a plurality of additional power modules;
each of the additional power modules includes additional set of stacks of solid oxide fuel cells, an additional anode tail gas oxidizer (ATO), and an additional exhaust conduit which is fluidly connected to the respective additional ATO and is configured to remove ATO exhaust from the respective additional power module;
the first branch fluidly further connects the splitter to the additional sets of stacks and is further configured to provide the first part of the first portion of the anode exhaust to the additional stacks; and
the second branch further fluidly connects the splitter to the additional ATOs and is further configured to provide the second part of the first portion of the anode exhaust to the additional ATOs.

9. A method, comprising:
providing fuel and air to a fuel cell system to generate electric power, an anode exhaust and a cathode exhaust;
separating at least a portion of water and carbon dioxide from the anode exhaust received from the fuel cell system to form syngas comprising at least 50 molar percent of a mixture of hydrogen and carbon monoxide;
recycling a first portion of the syngas output from the exhaust processing system to the fuel cell system; and
providing a second portion of the syngas output from the exhaust processing system to a syngas system to generate a product using the syngas.

10. The method of claim 9, wherein the syngas system comprises an iron ore reduction reactor which uses the syngas to reduce iron ore and generate the product comprising sponge iron.

11. The method of claim 9, wherein:
the syngas system comprises a catalytic reactor which performs perform a steam reformation process, an autothermal reformation process, or partial oxidation process to convert the syngas into the product comprising purified hydrogen; and
the syngas system further comprises a Haber reactor which converts the purified hydrogen and atmospheric nitrogen into ammonia using the Haber process.

12. The method of claim 9, wherein the syngas system comprises a Fischer-Tropsch reactor which converts the syngas into the product comprising liquid hydrocarbons using the Fischer-Tropsch process.

13. The method of claim 9, wherein the syngas system comprises a methanol reactor which converts syngas into the product comprising methanol.

14. The method of claim 9, further comprising providing the electric power from the fuel cell system to the syngas system, an electrical load, and an exhaust processing system which separates the water and the carbon dioxide from the anode exhaust,
wherein the step of separating the water and the carbon dioxide from the anode exhaust comprises:
condensing water from the anode exhaust; and
separating the carbon dioxide from the syngas in the anode exhaust after the condensing the water from the anode exhaust.

15. The method of claim 9, wherein:
the fuel cell system comprises a first powder module including a first set of stacks of solid oxide fuel cells, a first anode tail gas oxidizer (ATO), and a first exhaust conduit which is fluidly connected to the first ATO and removes ATO exhaust from the first power module;
a first part of the first portion of the anode exhaust is provided to the first stacks, and a second part of the first portion of the anode exhaust is provided to the first ATO;
the fuel cell system further comprises a plurality of additional power modules;
each of the additional power modules includes additional set of stacks of solid oxide fuel cells, an additional anode tail gas oxidizer (ATO), and an additional exhaust conduit which is fluidly connected to the respective additional ATO and is configured to remove ATO exhaust from the respective additional power module;
the first part of the first portion of the anode exhaust is further provided to the additional stacks;
the second part of the first portion of the anode exhaust is further provided to the additional ATOs; and
the method further comprises controlling the fuel cell system based on a flow rate and a composition of the fuel, and based on a flow rate and a composition of the first portion of the syngas.
